# EUROPÄISCHE PATENTSCHRIFT

(11) **EP 0 862 469 B1**
(45) Veröffentlichungstag und Bekanntmachung des Hinweises auf die Patenterteilung: **02.02.2000**
(21) Anmeldenummer: 96945872.8
(22) Anmeldetag: 08.11.1996
(51) Int. Cl.: A61M 3/02

(54) **VORRICHTUNG ZUM SPÜLEN VON KÖRPERHÖHLEN**
DEVICE FOR THE RINSING OF BODY CAVITIES
DISPOSITIF POUR RINCER DES CAVITES ORGANIQUES

(30) Priorität: 08.11.1995 DE 19541633
(43) Veröffentlichungstag der Anmeldung: 09.09.1998
(73) Patentinhaber: Storz Endoskop GmbH, 8200 Schaffhausen (CH)
(72) Erfinder: NOVAK, Paul, CH-8207 Schaffhausen (CH); HAMOU, Jacques, F-75016 Paris (FR); WATTIEZ, Arnaud, Ctr. Int. de Chirurgie Endoscopie, F-63000 Clermont-Ferrand (FR)
(74) Vertreter: Münich, Wilhelm, Dr.
(86) Internationale Anmeldenummer: DE9602138
(87) Internationale Veröffentlichungsnummer: WO9717093

(56) Entgegenhaltungen:
- WO-A-93/22979
- US-A- 4 261 360
- US-A- 5 178 606

## Beschreibung

### Technisches Gebiet

Die Erfindung betrifft eine Vorrichtung zum Spülen von Körperhöhlen mit einem Fluid gemäß dem Oberbegriff des Patentanspruchs 1.

Derartige Vorrichtungen werden beispielsweise von der Karl Storz GmbH & Co. unter den Warennamen "Hamou Endomat" oder "Uromat/Uropump" hergestellt und vertrieben und u.a. im Bereich der Gynäkologie ound der Urologie eingesetzt.

### Stand der Technik

Vor allem im Bereich der Gynäkologie und Urologie besteht die Gefahr der Intravasation (Einschwemmung), wenn während einer Dilatation eines behandelten Organs mit Spüllösung Gewebeabtragung vorgenommen wird. Dieses geschieht in der Regel mit einer Hochfrequenzschlinge z.B. in der Prostata oder bei der Gebärmutterschleimhaut.

Deshalb ist es notwendig, den intrauterinen bzw. intrauretalen Druck so zu wählen, daß eine ausreichende Dilatation und teilweise eine Blutstillung durch einen gewissen Überdruck an den Blutgefäßen erfolgt. Dieser Überdruck an den Blutgefäßen wird üblicherweise als "Abklemmen" bezeichnet.

Sofern jedoch beim Schneiden größere Blutgefäße verletzt werden, besteht die Gefahr, daß die Spülflüssigkeit in die Blutbahn eintritt. Dies kann bei Durchflußraten von mehreren 100 ml/min. im Betrieb des "continuous flow" leicht zu einem gefährlichen Einschwemmen der Spülflüssigkeit führen.

Dieses hätte ernste Konsequenzen für die Gesundheit des Patienten. Bei richtiger Handhabung von heutezutage verwendeten Spülpumpen, wie z.B. das Hamou-Endomat bzw. Uromat/Uropump, die über eine optimale Druck- und Flowregelung verfügen, kann dieses Risiko minimiert werden. Trotzdem muß das Bedienungspersonal bzw. der Arzt, der die Operation ausführt, zusätzlich während der Operation noch die Druck- und Flowregelung überwachen und optimieren. D.h., der behandelnde Arzt kann nicht mit voller Konzentration eine Operation durchführen. Zudem wäre es vor allem für weniger erfahrene Ärzte hilfreich, wenn sie eine zusätzliche Information zur weiteren Reduktion der Gefahr der Intravasation erhalten würden.

Die Firma Olympus Winter & Ibe bietet zur Zeit eine gattungsgemäße Spülpumpe an, die die Volumendifferenz zwischen verbrauchter Spüllösung und abgesaugter Spüllösung anzeigt. Hierbei wird das abgesaugte Volumen gravimetrisch und das verbrauchte Spülvolumen über die Drehzahl der Spülpumpe ermittelt.

Nachteilig bei diesem Gerät ist, daß die Volumendifferenz nur eine sehr unvollständige Information darüber liefert, ob eine Intravasation stattfindet. Hierbei ist insbesondere zu berücksichtigen, daß je nach Erfahrung des Operateurs mehr oder weniger viel Spülflüssigkeit durch Leckstellen oder z.B. durch das Herausziehen des Endoskops bzw. Resektoskops z.B. zwecks Reinigung verloren gehen kann.

Technisch bestünde die Möglichkeit, diese zusätzlichen Verluste durch ein zweites Auffanggefäß, das sich z.B. unterhalb des Patienten befindet, aufzufangen bzw. aufzusammeln. Dabei müßte eine Wiegezelle die zusätzlichen Verluste erfassen. Mit dem Ausgangssignal dieser Wiegezelle könnte das ermittelte Differenzvolumen in soweit korrigiert werden, daß allein nur die in den Körper gelangte Spülflüssigkeit angezeigt wird. Diese Möglichkeit birgt allerdings den Nachteil einer für den Anwender erschwerten Handhabung. Zudem besteht bei dieser Vorgehensweise die Gefahr, daß durch ungeschickte Handhabung der Instrumente eine gewisse Flüssigkeitsmenge nicht erfaßt wird; z.B. diejenige, die zwischen Körper und Instrument ausfließt. Zudem könnte bei einem Auffanggefäß, das unterhalb des Patienten angeordnet ist, durch eine unachtsame Belastung bzw. Entlastung des Auffanggefäßes z.B. ein zu kleiner Volumenverlust angezeigt werden.

Aus der nachveröffentlichten Offenlegungsschrift DE 44 17 189 Al der älteren deutschen Patentanmeldung P 44 17 189.7 ist ein Apparat zur Perfusion und Aspiration von (Spül)-Flüssigkeit in und aus Körperhöhlen bekannt, bei dem die Volumenströme erfaßt und die daraus gebildete Differenz zur Anzeige gebracht wird. Diese Vorgehensweise hat den Nachteil, daß immer nur Momentanwerte angezeigt werden, so daß der Operateuer nicht über die "Gesamt-Bilanz" der zugeführten und wieder abgeführten Spülflüssigkeit informiert wird.

Die US-A-5 178 606 offenbart den nächstliegenden Stand der Technik, bei dem eine elektronische Auswerteeinheit aus dem Ausgangssignal der Meßmittel die Menge des verlorenen Spülfluids bestimmt. Das heißt auch hier wird nur die Differenz zwischen dein Volumen der eingeleiteten Flüssigkeit und dem Volumen der aus den Körperhöhlen entfernten Flüssigkeit mit den schon oben aufgeführten Unzulänglichkeiten gemessen.

### Darstellung der Erfindung

Der Erfindung liegt die Aufgabe zugrunde, eine einfach handzuhabende Vorrichtung anzugeben, mit der die Überwachung der Intravasation auch für weniger erfahrene Ärzte optimal und zuverlässig dadurch erfolgt, daß der Arzt jederzeit über die gesamte bisherige Menge des verlorenen Spülfluids sowie die momentane Situation informiert ist,

Eine erfindungsgemäße Lösung dieser Aufgabe ist im Anspruch 1 angegeben. Weiterbildungen der Erfindung sind Gegenstand der Ansprüche 2 folgende.

Erfdinungsgemäß wird von einer Vorrichtung zum Spülen von Körperhöhlen mit einem Fluid ausgegangen, die ein Vorratsgefäß für ein Spülfluid, eine Pumpeneinheit, die das Spülfluid aus dem Vorratsgefäß über ein Instrument über die zu behandelnde Körperhöhle pumpt, einen Auffangbehälter für das aus der Körperhöhle abfließende Spülfluid und Meßmittel aufweist, die die Differenz zwischen eingebrachtem Spülfluid und abfliessendem bzw. abgesaugtem Spülfluid ermitteln.

Erfindungsgemäß weist die Vorrichtung eine elektronische Auswerteeinheit auf, die aus dem Ausgangssignal der Meßmittel die Menge des verlorenen Spülfluids und zusätzlich die zeitliche Rate bestimmt, mit der sich die Verlustmenge ändert. Erfindungsgemäß wird also das durch den Stand der Technik aufgezeigte Problem dadurch gelöst, daß bei einer gattungsgemäßen Vorrichtung die Volumendifferenz zwischen verbrauchter und abgesaugter Spüllösung ermittelt und insbesondere angezeigt wird, und daß zusätzlich die Rate, mit der sich dieses Differenzvolumen ändert, ermittelt und beispielsweise wahlweise angezeigt wird (vgl. auch Ansprüche 11 und 12).

Ausdrücklich soll darauf hingewiesen werden, daß die Differenz zwischen zugeführtem und abfliessendem Fluid nicht nur negativ, sondern aufgrund von zusätzlich abgezogenen Körper-Fluiden, wie Blut auch positiv sein kann.

Vorteilhafterweise saugt die Pumpeneinheit gemäß Anspruch 2 zusätzlich das Spülfluid aus der Körperhöhle ab. Durch diese Maßnahme wird ein definierterer (Rück)-Fluß der Spülflüssigkeit gewährleistet.

Gemäß Anspruch 3 kann die Pumpeneinheit zwei Pumpen aufweisen, mit denen es möglich ist, die Flußraten des einzubringenden Spülfluids und des abgesaugten Spülfluids individuell zu steuern bzw. zu regeln (Anspruch 10).

Prinzipiell können die Meßmittel auf die verschiedensten Arten ausgebildet sein und die unterschiedlichsten Größen ermitteln, solange diese es nur gestatten, die Menge des verlorenen Spülfluids und zusätzlich die zeitliche Rate zu bestimmen, mit der sich die Verlustmenge ändert.

Im Anspruch 4 ist eine besonders vorteilhafte Weiterbildung angegeben, bei der die Meßmittel das Gewicht des Vorratsgefäßes und des Auffangbehälters ermitteln. Dies ermöglicht nicht nur eine einfache und kompakte Bauweise insbesondere der Meßmittel, sondern gestattet auch eine genaue Ermittlung der einzelnen Meßwerte.

Insbesondere ist es gemäß Anspruch 5 bevorzugt, daß die Meßmittel nur eine einzige Wiegezelle aufweisen, die sowohl mit dem Gewicht des Vorratsgefäßes als auch mit dem Gewicht des Auffangbehälters beaufschlagt wird. Hierdurch wird nicht nur ein einfacher Aufbau der erfindungsgemäßen Vorrichtung erreicht, sondern auch die Meßgenauigkeit erhöht, das lediglich eine Änderung gegenüber einem austarierbaren Wert festgestellt werden muß. Dabei ist es gemäß Anspruch 6 von Vorteil, wenn das Vorratsgefäß und der Auffangbehälter an der Wiegezelle hängen, da hierdurch der Platzbedarf weiter verringert wird.

Natürlich ist es auch möglich, zwei Waagen als Meßmittel vorzusehen.

Zusätzlich wird vorteilshafterweise gemäß Anspruch 7 bei Überschreiten eines bestimmten Wertes bzw. einer bestimmten zeitlichen Rate der Verlustmenge ein Warnsignal in Form eines akustischen und/oder optischen Alarms ausgelöst. Ein derartiges Warnsignal kann z.B. dann ausgelöst werden, wenn ein Spülflüssigkeitsfluß ermittelt worden ist, der voll in die Blutbahn des Patienten gelangt, oder aber einem Fluß entspricht, der z.B. während der Reinigung eines Resektoskops im Freien landet. Im Falle eines Alarms ist für den Anwender bzw. den Operateur lediglich zu entscheiden, ob eine rasch ansteigende Zunahme der Volumendifferenz eine Intravasation bedeutet oder lediglich einen handhabungsbedingten Flüssigkeitsverlust darstellt.

Da die kritische Rate der (negativen bzw. positiven) Änderung des Gewichts des Auffangbehälters und des Vorratsgefäßes auch von der gewählten Operationstechnik abhängt, ist gemäß Anspruch 8 die Änderungs-Rate, bei der ein Alarm ausgelöst wird, vom Benutzer einstellbar ist.

Die erfindungsgemäß vorgesehene Auswerteeinheit kann nicht nur dazu eingesetzt werden, dem Arzt die Meßwerte auf einer Anzeigeeinheit darzustellen, sondern auch dazu, die Pumpeneinheit zu steuern (Anspruch 9).

Insbesondere ist es gemäß Anspruch 10 möglich, daß die Auswerteeinheit die Pumpeneinheit aufgrund des Ausgangssignals des Meßmittels derart regelt, daß der Fluß des Spülfluids aus dem Vorratsgefäß und der Fluß des Spülfluids in den Auffangbehälter in etwa gleich sind.

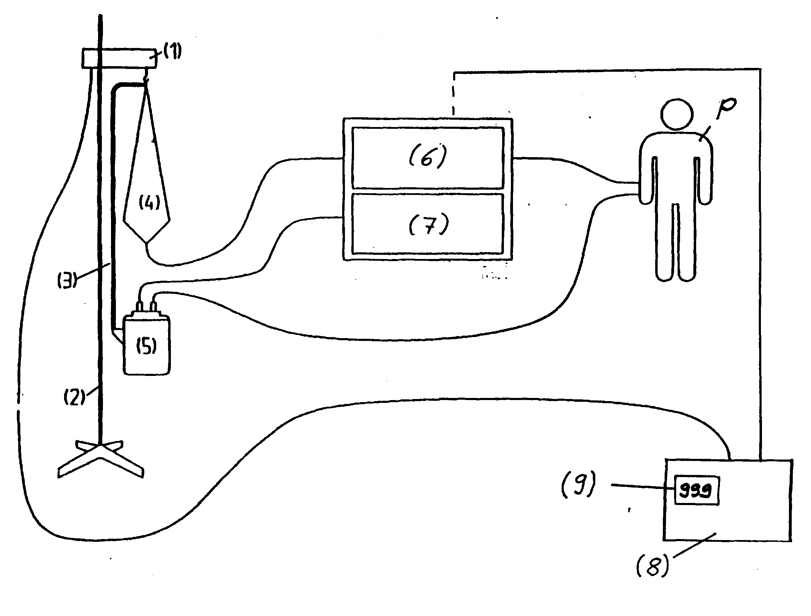

### Kurze Beschreibung der Zeichnung

Die Erfindung wird nachstehend anhand eines Ausführungsbeispiels unter Bezugnahme auf die Zeichnung näher erläutert, deren
einzige Figur schematisch eine erfindungsgemäße Vorrichtung zeigt.

### Beschreibung eines Ausführungsbeispiels

Die erfindungsgemäße Vorrichtung zum Spülen einer Körperhöhle eines Patienten P mit einem Fluid weist eine Wiegezelle 1 auf, die an einem Stativ 2 befestigt ist. An der Wiegezelle 1 ist ein Bügel 3 angebracht, an dem ein Vorratsgefäß 4 für das Spülfluid und ein Auffangbehälter 5 für das aus der Körperhöhle abfließende Spülfluid hängend angebracht sind. Die Wiegezelle 1 erfaßt damit das Gesamtgewicht der Behälter 4 und 5.

Das Vorratsgefäß 4 ist über eine Leitung mit einer Spülpumpe 6 einer Pumpeneinheit verbunden, die das Spülfluid aus dem Vorratsgefäß 4 über ein nicht dargestellten Instrument in die zu behandelnde Höhle des Körpers P pumpt,

Ferner weist die Pumpeneinheit eine Saugpumpe 7 auf, die das Spülfluid über eine Leitung aus der Körperhöhle in den Auffangbehälter 5 absaugt.

Die Wiegezelle 1 erfaßt fortlaufend das Gesamtgewicht der beiden Behälter 4 und 5. Das Ausgangssignal der Wiegezelle 1 ist über eine Leitung an einer elektronischen Auswerteeinheit 8 angelegt. Die Auswerteeinheit 8 ermittelt aus dem das Gesamtgewicht angegebenden Ausgangssignal die Differenz zwischen eingebrachtem Spülfluid und abfließendem Spülfluid und bestimmt hieraus die Menge des verlorenen Spülfluids und zusätzlich die zeitliche Rate, mit der sich die Verlust-Menge ändert. Die derart bestimmten Werte können auf einer Anzeigeeinheit 9 alternierend oder gleichzeitig dargestellt werden. Zusätzlich ist ein optischer und/oder akustischer Alarm vorgesehen, der bei Überschreiten einer bestimmten Änderungs-Rate der Verlustmenge ausgelöst wird.

Zusätzlich kann vorgesehen sein, daß die Auswerteeinheit 8 die Pumpen 6 und 7 aufgrund des Ausgangssignals der Wiegezelle 1 derart regelt, daß der Fluß des Spülfluids aus dem Vorratsgefäß 4 und der Fluß des Spülfluids in den Auffangbehälter 5 in etwa gleich sind.

## Patentansprüche

1. Vorrichtung zum Spülen von Körperhöhlen mit einem Fluid, mit
- einem Vorratsgefäß (4) für das Spülfluid,
- einer Pumpeneinheit, die das Spülfluid aus dem Vorratsgefäß über ein Instrument in die zu behandelnde Körperhöhle pumpt,
- einem Auffangbehälter (5) für das aus der Körperhöhle abfließende Spülfluid,
- Meßmitteln, die die Differenz zwischen eingebrachtem Spülfluid und abfließendem Spülfluid ermitteln,
- und einer elektronischen Auswerteeinheit (8) zur aus dem Ausgangssignal der Meßmittel erfolgenden Bestimmung der Menge des verlorenen Spülfluids,
dadurch **gekennzeichnet,** daß die elektronische Auswerteeinheit (8) aus dem Ausgangssignal der Meßmittel zusätzlich die zeitliche Rate bestimmt, mit der sich die Verlust-Menge ändert.

2. Vorrichtung nach Anspruch 1,
dadurch **gekennzeichnet,** daß die Pumpeneinheit auch das Spülfluid aus der Körperhöhle absaugt.

3. Vorrichtung nach Anspruch 2,
dadurch **gekennzeichnet,** daß die Pumpeneinheit zwei Pumpen aufweist, von denen die eine Pumpe (6) das Spülfluid aus dem Vorratsgefäß (4) über das Instrument in die zu behandelnde Körperhöhle pumpt, und die andere Pumpe (7) das Spülfluid aus der Körperhöhle absaugt.

4. Vorrichtung nach einem der Ansprüche 1 bis 3,
dadurch **gekennzeichnet,** daß die Meßmittel das Gewicht des Vorratsgefäßes (4) und des Auffangbehälters (5) ermitteln.

5. Vorrichtung nach Anspruch 4,
dadurch **gekennzeichnet,** daß die Meßmittel eine einzige Wiegezelle (1) aufweisen, die sowohl mit dem Gewicht des Vorratsgefäßes (4) als auch mit dem Gewicht des Auffangbehälters (5) beaufschlagt wird.

6. Vorrichtung nach Anspruch 5,
dadurch **gekennzeichnet,** daß das Vorratsgefäß (4) und der Auffangbehälter (5) an der Wiegezelle (1) hängen.

7. Vorrichtung nach einem der Ansprüche 1 bis 6,
dadurch **gekennzeichnet,** daß die Auswerteeinheit (8) bei Überschreiten einer bestimmten Änderungs-Rate der Verlust-menge einen akustischen und/oder optischen Alarm auslöst.

8. Vorrichtung nach Anspruch 7,
dadurch **gekennzeichnet,** daß die Änderungs-Rate, bei der ein Alarm ausgelöst wird, vom Benutzer einstellbar ist.

9. Vorrichtung nach einem der Ansprüche 1 bis 8,
dadurch **gekennzeichnet,** daß die Auswerteeinheit (8) die Pumpeneinheit steuert.

10. Vorrichtung nach Anspruch 9,
dadurch **gekennzeichnet,** daß die Auswerteeinheit (8) die Pumpeneinheit aufgrund des Ausgangssignals des Meßmittels derart regelt, daß der Fluß des Spülfluids aus dem Vorratsgefäß (4) und der Fluß des Spülfluids in den Auffangbehälter (5) gleich sind.

11. Vorrichtung nach einem der Ansprüche 1 bis 10,
dadurch **gekennzeichnet,** daß das Ausgangssignal der Auswerteeinheit (8) an eine Anzeigeeinheit (9) angelegt ist, die die Menge des verlorenen Spülfluids und zusätzlich die zeitliche Rate anzeigt, mit der sich die Verlust-Menge ändert.

12. Vorrichtung nach Anspruch 11,
dadurch **gekennzeichnet,** daß die Anzeigeeinheit (9) von der Anzeige der Menge des verlorenen Spülfluids auf die Anzeige der zeitlichen Rate, mit der sich die Verlust-Menge ändert, umschalbar ist.

## Claims

1. Apparatus for rinsing body cavities with a fluid, comprising
- a reservoir (4) containing the irrigating fluid,
- a pumping unit which pumps the irrigating fluid from said reservoir via an instrument into the body cavity to be treated,
- a collector vessel (5) receiving the irrigating fluid flowing out of the body cavity,
- measuring means which detect the difference between the introduced quantity of irrigating fluid and the flowing-out quantity of irrigating fluid,
- and an electronic evaluating unit (8) for detecting the quantity of irrigating fluid lost on the basis of the output signal from said measuring means,
**characterized in** that said electronic evaluation unit (8) determines additionally the rate of variation of the lost quantity versus time.

2. Apparatus according to Claim 1,
**characterized in** that said pumping unit also aspirates the irrigating fluid from the body cavity.

3. Apparatus according to Claim 2,
**characterized** in that said pumping unit includes two pumps whereof one (6) pumps the irrigating fluid from said reservoir (4) via the instrument into the body cavity to be treated, whilst the other one (7) aspirates the irrigating fluid from the body cavity.

4. Apparatus according to any of the Claims 1 to 3,
**characterized in** that said measuring means detect the weight of said reservoir (4) and of said collector vessel (5).

5. Apparatus according to Claim 4,
**characterized in** that said measuring means include a single weighing cell (1) loaded with both the weight of said reservoir (4) and the weight of said collector vessel (5).

6. Apparatus according to Claim 5,
**characterized in** that said reservoir (4) and said collector vessel (5) are suspended on said weighing cell (1).

7. Apparatus according to any of the Claims 1 to 6,
**characterized in** that said evaluation unit (8) triggers an acoustic and/optical alarm when a defined rate of variation of the lost quantity is exceeded.

8. Apparatus according to Claim 7,
**characterized in** that the rate of variation at which an alarm is triggered may be set by the user.

9. Apparatus according to any of the Claims 1 to 8,
**characterized in** that said evaluation unit (8) controls said pumping unit.

10. Apparatus according to Claim 9,
**characterized in** that said evaluation unit (8) controls said pumping unit on the basis of the output signal of said measuring means in a way that the flow of the irrigating fluid from the reservoir (4) and the flow of the irrigating fluid into said collector vessel (5) are approximately equal.

11. Apparatus according to any of the Claims 1 to 10,
**characterized in** that the output signal of said evaluation unit (8) is applied to a display unit (9) which indicates the quantity of lost irrigating fluid and additionally the rate of variation of the lost quantity versus time.

12. Apparatus according to Claim 11,
**characterized in** that said display unit (9) may be switched over from the indication of the quantity of lost irrigating fluid to the indication of the rate of variation of the lost quantity versus time.

## Revendications

1. Dispositif de rinçage de cavités corporelles à l'aide d'un fluide, comprenant
- un récipient de réserve (4) pour le fluide de rinçage,
- une unité de pompage qui pompe le fluide de rinçage hors du récipient de réserve, par l'intermédiaire d'un instrument, jusque dans la cavité corporelle devant être traitée,
- un réceptacle (5) pour le fluide de rinçage s'écoulant hors de la cavité corporelle,
- des moyens mesureurs établissant la différence entre du fluide de rinçage introduit et du fluide de rinçage s'écoulant,
- et une unité d'interprétation électronique (8) en vue de la détermination, s'opérant sur la base du signal de sortie des moyens mesureurs, de la quantité du fluide de rinçage perdu,
caractérisé par le fait que l'unité d'interprétation électronique (8) détermine additionnellement, sur la base du signal de sortie des moyens mesureurs, l'allure temporelle selon laquelle la quantité perdue varie.

2. Dispositif selon la revendication 1,
caractérisé par le fait que l'unité de pompage aspire également le fluide de rinçage hors de la cavité corporelle.

3. Dispositif selon la revendication 2,
caractérisé par le fait que l'unité de pompage comprend deux pompes parmi lesquelles l'une (6) pompe le fluide de rinçage à partir du récipient de réserve (4), par l'intermédiaire de l'instrument, jusque dans la cavité corporelle devant être traitée, et l'autre pompe (7) aspire le fluide de rinçage hors de ladite cavité corporelle.

4. Dispositif selon l'une des revendications 1 à 3,
caractérisé par le fait que les moyens mesureurs établissent le poids du récipient de réserve (4) et du réceptacle (5).

5. Dispositif selon la revendication 4,
caractérisé par le fait que les moyens mesureurs présentent une unique cellule de pesage (1) sollicitée aussi bien par le poids du récipient de réserve (4), que par le poids du réceptacle (5).

6. Dispositif selon la revendication 5,
caractérisé par le fait que le récipient de réserve (4) et le réceptacle (5) sont suspendus à la cellule de pesage (1).

7. Dispositif selon l'une des revendications 1 à 6,
caractérisé par le fait que l'unité d'interprétation (8) déclenche une alarme acoustique et/ou optique en cas de dépassement d'une allure de variation déterminée de la quantité perdue.

8. Dispositif selon la revendication 7,
caractérisé par le fait que l'allure de variation, en présence de laquelle une alarme est déclenchée, peut être réglée par l'utilisateur.

9. Dispositif selon l'une des revendications 1 à 8,
caractérisé par le fait que l'unité d'interprétation (8) commande l'unité de pompage.

10. Dispositif selon la revendication 9,
caractérisé par le fait que l'unité d'interprétation (8) régule l'unité de pompage, sur la base du signal de sortie du moyen mesureur, de façon telle que l'écoulement du fluide de rinçage hors du récipient de réserve (4), et l'écoulement dudit fluide de rinçage dans le réceptacle (5), soient identiques.

11. Dispositif selon l'une des revendications 1 à 10,
caractérisé par le fait que le signal de sortie de l'unité d'interprétation (8) est appliqué à une unité d'affichage (9) qui affiche la quantité du fluide de rinçage perdu et, additionnellement, l'allure temporelle selon laquelle la quantité perdue varie.

12. Dispositif selon la revendication 11,
caractérisé par le fait que l'unité d'affichage (9) peut être commutée pour passer de l'affichage de la quantité du fluide de rinçage perdu, à l'affichage de l'allure temporelle selon laquelle la quantité perdue varie.
